# EUROPÄISCHE PATENTSCHRIFT

(11) **EP 1 154 976 B1**
(45) Veröffentlichungstag und Bekanntmachung des Hinweises auf die Patenterteilung: **10.09.2003**
(21) Anmeldenummer: 00907632.4
(22) Anmeldetag: 25.02.2000
(51) Int. Cl.: C07C 47/02, C07C 45/50

(54) **VERFAHREN ZUR HYDROFORMYLIERUNG VON OLEFINEN**
METHOD FOR THE HYDROFORMYLATION OF OLEFINS
PROCEDE D'HYDROFORMYLATION D'OLEFINES

(30) Priorität: 26.02.1999 DE 19908462
(43) Veröffentlichungstag der Anmeldung: 21.11.2001
(73) Patentinhaber: BASF AKTIENGESELLSCHAFT, 67056 Ludwigshafen (DE)
(72) Erfinder: ZELLER, Edgar, D-68165 Mannheim (DE); RÖPER, Michael, D-67157 Wachenheim (DE); DECKER, Jürgen, D-67346 Speyer (DE)
(74) Vertreter: Kinzebach, Werner, Dr.
(86) Internationale Anmeldenummer: EP0001585
(87) Internationale Veröffentlichungsnummer: WO00050373

(56) Entgegenhaltungen:
- WO-A-99/36382
- DE-A- 19 603 201
- US-A- 4 845 306
- CHEMICAL ABSTRACTS, Band 117, Nr. 9, 31 August 1992, Columbus, Ohio, US, Zusammenfassungs-Nr. 89808z, K.KANEDA ET AL:"New polymer- -bound rhodium carbonyl cluster catalysts containing two functional ligands for hydrohydroxy-methylation of olefins", Seite 735, Spalte 1, XP002901061 & J.Mol.Catal. 1992, 72(3) L27-L30 (Eng),
- CHEMICAL ABSTRACTS, Band 80, Nr. 19, 13 Mai 1974 Columbus, Ohio, US, Zusammenfassungs-Nr. 107936p, W.O. HAAG ET AL:"New hetero- -geneous oxo catalyst for liquid phase processes", Seite 367, Spalte 2, XP002901062 & Catal.,Proc.Int. Congr., 5th 1972 (Pub.1973), 1, 465-75 (Eng),
- PATENT ABSTRACTS OF JAPAN Band 4, Nr. 12 (C-71), 29 Januar 1980; & JP 54 148710 A (TOA NENRYO KOGYO KK.) 21 November 1979,

## Beschreibung

Die vorliegende Erfindung betrifft ein Verfahren zur Hydroformylierung von Olefinen mit wenigstens 7 Kohlenstoffatomen. Die Hydroformylierung höherer Olefine, d.h. solcher mit wenigstens 7 Kohlenstoffatomen, besitzt große wirtschaftliche Bedeutung. Die Hydroformylierung von Olefinen mit Kohlenmonoxid und Wasserstoff verläuft in Gegenwart von homogen im Reaktionsmedium verteilten Übergangsmetall-Katalysatoren. Im Anschluß an die Hydroformylierung muß der Katalysator von den Hydroformylierungsprodukten abgetrennt werden. Während bei der technisch überwiegend ausgeübten Cobalt-katalysierten Hydroformylierung bereits viele Lösungen zur Katalysatorabtrennung vorliegen (vgl. J. Falbe, Hrsg.: New Synthesis with Carbon Monoxide, Springer, Berlin, 1980, S. 55 ff) ist die Abtrennung von Rhodium-Katalysatoren von den Hydroformylierungsprodukten bei der Synthese höherer Aldehyde technisch noch nicht zufriedenstellend gelöst.

Die DE 198 01 437.6 beschreibt ein Hydroformylierungsverfahren unter Verwendung eines homogen im Reaktionsgemisch gelösten Komplexkatalysators, der ein Metall der Gruppe VIII des Periodensystems und ein Polyamin mit mindestens 10 Stickstoffatomen und einem Molekulargewicht von größer 1000 Dalton enthält. Aus dem Reaktionsgemisch werden nach beendeter Hydroformylierung die gebildeten Aldehyde und Alkohole destillativ entfernt, wobei der Kata) lysatorkomplex im Destillationssumpf zurückbleibt.

Während bei der Umsetzung niedriger Olefine die thermische Abtrennung der Hydroformylierungsprodukte z.B. destillativ meist problemlos erfolgen kann, muß bei höheren Olefinen zum Zweck der Destillation eine hohe Temperatur eingestellt werden, wodurch der Rhodium-Katalysator geschädigt werden kann. Der thermolabile Rhodium-Katalysator kann sich z.B. infolge der thermischen Belastung bei der destillativen Aufarbeitung des Hydroformylierungsproduktes teilweise zu metallischem Rhodium zersetzen, das sich an den Wandungen des Reaktors und den Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Damit verbundene Rhodiumverluste haben bislang eine größere industrielle Anwendung der Rhodium-katalysierten Hydroformylierung zur Herstellung höherer Aldehyde verhindert. Gleichzeitig oder alternativ muß bei der Destillation ein hohes Vakuum erzeugt werden, was bei großtechnisch hergestellten Produkten hohe Investition- und Betriebskosten bedingt. Bei noch höheren Aldehydprodukten, z.B. Polymeren, die nicht destilliert werden können, ist eine destillative Produktabtrennung nicht möglich.

Zur Lösung dieser Probleme sind in der Literatur zahlreiche Verfahren vorgeschlagen worden. So schlägt die DE- A 4230871 eine Extraktion des Katalysators mit sulfonierten, stickstoffhaltigen niedermolekularen Komplexbildnern in eine wässrige Phase vor. Der wässrige Extrakt des Hydroformylierungsaustrags, welcher den nunmehr vom wasserlöslichen Polymer komplexierten Rhodium-Katalysator enthält, wird einer Vorcarbonylierung zugeleitet, in der der komplexierte Rhodium-Katalysator in Gegenwart einer im Wesentlichen wasserunlöslichen organischen Flüssigkeit und in Gegenwart von Kohlenmonoxid, Synthesegas oder eines Kohlenmonoxid-haltigen Gasgemisches carbonyliert wird. Durch die Carbonylierung wird das Rhodium aus dem hydrophilen Komplex mit dem wasserlöslichen Polymer gelöst, und die sich dabei bildende, lipophile Rhodium-Carbonylverbindung migriert in die wasserunlösliche organische Flüssigkeit. Dieser Schritt wird als "Vorcarbonylierung" bezeichnet, da die Carbonylierung des Rhodiums nicht im Hydroformylierungsreaktor selbst, sondern in der vorgeschalteten Vorcarbonylierungsstufe erfolgt.

Die WO 97/30016 schlägt ein ähnliches Verfahren mit Extraktion des Katalysators aus dem Hydroformylierungsaustrag mit einer wässrigen Lösung eines sulfonsäuregruppenfreien, wasserlöslichen zur Komplexbildung von Rhodium befähigten Polymeren vor, wobei als wasserlösliche Polymere vorzugsweise Polyacrylsäure, Maleinsäure-Copolymere, phosphonomethylierte Polyvinylamine, phosphonomethylierte Polyethylenimine und/oder phosphonomethylierte Polyacrylamide eingesetzt werden. Den Verfahren aus den beiden vorgenannten Druckschriften ist gemeinsam, dass die polymeren Komplexbildner nicht in der Reaktionszone vorliegen, sondern lediglich zur Behandlung des Reaktionsaustrages zugefügt werden. Nachteilig ist bei den beschiebenen Verfahren, dass mehrfache Extraktionsschritte notwendig sind.

Die EP 711 748 beschreibt ein Verfahren zur Herstellung von Formylcarbonsäureestern durch Hydroformylierung von mehrfach ungesättigten Fettsäureestern in Gegenwart einer wässrigen Lösung, die Rhodium-Phosphin-Komplexverbindungen als Katalysatoren und weiterhin einen Phasentransferkatalysator, z.B. quaternäre Ammoniumverbindungen, enthält. Hierbei wird jedoch nachteiligerweise neben dem Katalysatormetall, dem Liganden und dem Produkt ein weiterer Stoff in Form des Phasentransferkatalysator in das Reaktionsgemisch eingebracht. Die Abtrennung des Phasentransferkatalysator vom Hydroformylierungsprodukt, bzw. vom Katalysator bereitet jedoch aufgrund der Amphiphilie des Phasentransferkatalysators Probleme.

Die EP 0633 062 schlägt den Einsatz von fluorierten Lösungsmitteln in Verbindung mit fluorierten Liganden vor, um Hydroformylierungsprodukte und Katalysator abtrennbar zu machen. Nachteilig sind an diesem Vorschlag der hohe Preis der fluorierten Lösungsmittel, die umständlichen Synthesen der Liganden und die nachteilige Handhabung von Fluor. Bei den durch Beispiele veranschaulichten Liganden handelt es sich um Phosphanderivate.

Die Literaturstelle Abatioglu et al., Catalysis of Organic Reactions, Chem. Ind. (Dekker) 68 (1996) S.133-39 beschreibt die Hydroformylierung unter Verwendung von Rhodium-Katalysatoren mit ionischen Phosphinkatalysatoren. Diese bilden in Gegenwart von Lösungsmitteln wie N-Methylpyrrolidon eine einheitliche Phase mit den unpolaren Hydroformylierungsedukten und -produkten. Nach Zugabe von Wasser zerfällt das Reaktionsgemisch in zwei Phasen, und die Rhodium-Phosphin-haltige N-Methylpyrrolidon-Phase kann vom Produkt abgetrennt werden.

Fell et al., J. f.prakt.Chem., 338 (1996) S.124-28 und J. of Mol. Catalysis A: Chemical 116, (1997) S. 55-58 beschreiben wasserlösliche polyethersubstituierte Triphenylphosphine, die eine temperaturabhängige Wasserlöslichkeit aufweisen. Komplexe der polyethersubstituierten Triphenylphosphine mit Rhodium werden zur Katalyse der Hydroformylierung höherer Olefine in Gegenwart von Wasser verwendet. Bei geeignet hohen Reaktionsbedingungen bildet sich eine einheitliche Phase, die beim Abkühlen auf Raumtemperatur in zwei Phasen zerfällt.

Die vorstehenden bekannten Verfahren sind durch die Verwendung phosphinmodifizierter Katalysatoren gekennzeichnet. Während α-Olefine sehr gut mit rhodiumhaltigen phosphinmodifizierten Katalysatoren hydroformylierbar sind, sind derartige Katalysatorsysteme für die Hydroformylierung interner Olefine und an der Doppelbindung verzweigter Olefine, sowie interner und verzweigter Olefine wenig geeignet (vgl. Falbe, loc. cit., S.95 ff). So werden interne Kohlenstoff-Kohlenstoff-Doppelbindungen sehr langsam in Gegenwart eines derartigen Katalysators hydroformyliert. Großtechnisch zugängliche höhere Olefine enthalten meist einen beträchtlichen Anteil interner und/oder verzweigter Olefine. Beispiele hierfür sind Oligomere aus Propen oder Butenen, z.B. aus Dimersol-Verfahren, interne lineare Olefine, z.B. aus dem SHOP-Prozess oder der Paraffin-Dehydrierung, die wichtige Ausgangsstoffe zur Synthese von Weichmacher- und Tensid-Alkoholen sind. Ferner können auch α-Olefine aus z.B. Ziegler-Verfahren einen gewissen Anteil an internen oder verzweigten Olefinen enthalten, z.B. durch die im Prozess auftretende Co-Oligomerisierung von höheren Olefinen, wie 1-Buten oder 1-Hexen.

Aus der US 4448 996 geht ein Verfahren zur Herstellung von tertiären Aminen durch Umsetzung langkettiger Olefine mit Kohlenmonoxid, Wasserstoff und einem primären oder sekundären Amin in Gegenwart eines Rhodium- und/oder Rutheniumhaltigen Katalysators unter Verwendung bestimmter Lösungsmittel hervor, wobei die Reaktionsmischung nach der Umsetzung in eine Produktphase und eine katalysatorhaltige Lösungsmittelphase zerfällt. Als die Phasentrennung bewirkende Lösungsmittel sind z.B. Alkohole und Glycole geeignet.

Der vorliegenden Erfindung liegt die Aufgabe zugrunde, ein Verfahren zur Hydroformylierung von Olefinen mit wenigstens 7 Kohlenstoffatomen anzugeben, das für α-Olefine und interne. Olefine, verzweigte Olefine und interne verzweigte Olefine gleichermaßen geeignet ist und eine einfache Abtrennung des Katalysators von den Hydroformylierungsprodukten gestattet.

Erfindungsgemäß wird diese Aufgabe durch ein Verfahren gelöst, bei dem man a) das Olefin mit Kohlenmonoxid und Wasserstoff in Gegenwart eines Komplexes von Rhodium und eines polymeren Komplexliganden, der mehr als 10 Stickstoffatome enthält und eine Molmasse von mehr als 1000 g/mol aufweist, sowie eines Solubilisierungsmittels bei einer Temperatur von 40 bis 200°C und einem Druck von 1 bis 600 bar in einer Reaktionszone einer Hydroformylierungsreaktion unterwirft, wobei unter den Reaktionsbedingungen das Solubilisierungsmittel mit dem Olefin und dem gebildeten Hydroformylierungsprodukt eine weitgehend einheitliche Phase bildet, in welcher der Komplex solubilisiert ist, b) in dem Reaktionsgemisch eine Phasentrennung in eine solubilisierungsmittelreiche Phase und eine solubilisierungsmittelarme Phase induziert, wobei der Komplex bevorzugt in der solubilisierungsmittelreichen Phase vorliegt und das Hydroformylierungsprodukt bevorzugt in der solubilisierungsmittelarmen Phase vorliegt, und c) die Phasen voneinander abtrennt. Vorzugsweise liegen in der Reaktionszone weniger als 10 Gew.-%, insbesondere weniger als 5 Gew.-%, bezogen auf das Reaktionsgemisch, Wasser vor. Bei dem Hydroformylierungsprodukt kann es sich um eine einzelne chemische Verbindung oder um ein Produktgemisch handeln.

In einer bevorzugten Ausführungsform wird die solubilisierungsmittelreiche Phase, gegebenenfalls nach weiterer Behandlung, ganz oder teilweise in die Reaktionszone zurückgeführt.

Es wurde überraschenderweise gefunden, dass bestimmte polymere stickstoffhaltige Liganden vorteilhafte Komplexierungseigenschaften für Rhodium zeigen und Rhodium-Katalysatoren hoher Hydroformylierungsaktivität bilden. Die Mitverwendung eines Solubilisierungsmittels gestattet eine Solubilisierung des Katalysators in der Reaktionszone unter Reaktionsbedingungen und eine einfache Katalysatorabtrennung nach beendeter Hydroformylierungsreaktion durch Bildung einer Produktphase und einer die Hauptmenge des Katalysators enthaltenden Phase. "Solubilisiert" bedeutet für die Zwecke der vorliegenden Erfindung, dass der Komplex hinreichend homogen verteilt im Reaktionsgemisch ist, um die Hydroformylierungsreaktion zu katalysieren. Im Allgemeinen ist der Komplex entweder homogen gelöst oder zumindest kolloidal verteilt.

Als Komplexliganden werden polymere Liganden, die mehr als 10 Stickstoffatome enthalten und eine Molmasse von mehr als 1000 g/mol, vorzugsweise mehr als 10 000 bis 10⁶ g/mol aufweisen, verwendet. Bei dem polymeren stickstoffhaltigen Liganden handelt es sich im Allgemeinen um Homopolymere oder Copolymere stickstoffhaltiger Monomereinheiten. Geeignete Beispiele sind Polyalkylenimine, insbesondere Polyethylenimine; Polyvinylamine; Polymere ethylenisch ungesättigter Carbonsäureamide, wie Poly(meth)acrylamide; Polymere acyclischer oder cyclischer N-Vinylamide, wie Polyvinylformamid, Polyvinylpyrrolidon oder Polyvinylcaprolactam; Polymere vinyl- oder allylsubstituierter stickstoffhaltiger Heteroaromaten, wie Polyvinylpyridin. Die Polymere können in einem Molekül unterschiedliche stickstoffhaltige Monomere und gegebenenfalls stickstofffreie Monomere enthalten. Die Stickstoffatome können sich in der Hauptkette befinden oder in Seitengruppen vorliegen.

Die Polarität des stickstoffhaltigen polymeren Liganden ist so gewählt, daß dieser unter den Reaktionsbedingungen im Reaktionsgemisch solubilisiert ist und nach der Phasentrennung bevorzugt in der solubilisierungsmittelreichen Phase vorliegt. In der Regel sind die polymeren stickstoffhaltigen Liganden zur Einstellung einer geeigneten Polarität derivatisiert. Im Fall aminogruppenhaltiger Polymere tragen diese z.B. an einem Teil der oder allen Aminogruppen Substituenten, wie Alkyl-, Aryl-, Acyl- oder Polyoxyalkylengruppen. Die Einführung von Substituenten kann durch Derivatisierung mit geeigneten Derivatisierungsreagenzien, wie Carbonsäuren, Carbonsäurederivaten, Alkylierungsmitteln oder Alkenoxiden, durch Phosphonomethylierung, Strecker-Synthese usw. erfolgen. Die Derivatisierung kann an Stickstoffatomen oder an anderen Positionen des Polymeren erfolgen. Die Einführung funktioneller Gruppen kann durch polymeranaloge Umsetzung des stickstoffhaltigen Polymeren oder auf der Stufe der zugrundeliegenden Monomeren oder durch Mitverwendung geeigneter copolymerisierbarer stickstofffreier Monomere erfolgen.

Als Liganden brauchbar sind Polyethylenimine, bestehend im Wesentlichen aus Einheiten der Formel I oder verzweigte Isomere davon, 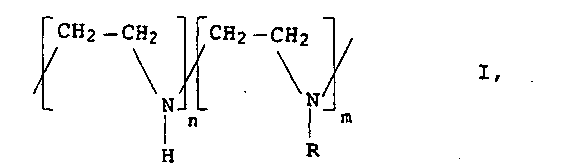 in der die Summe aus m + n mindestens 10 und das Verhältnis aus m/m+n 0,01 bis 1 beträgt und R gleiche oder verschiedene Alkyl-, Cycloalkyl-, Aryl-, Aralkyl-, aliphatische, araliphatische oder aromatische Acylreste mit bis zu 30 C-Atomen oder Hydroxyalkyl(poly)oxyalkylenreste mit bis zu 500 Alkylenoxy-Einheiten, vorzugsweise mit 2 bis 6 C-Atomen pro Alkylenoxy-Einheit, bedeuten.

Als erfindungsgemäß zu verwendende Liganden kommen insbesondere Polyethylenimine bestehend im wesentlichen aus Einheiten der Formel II oder verzweigte Isomere davon in Betracht 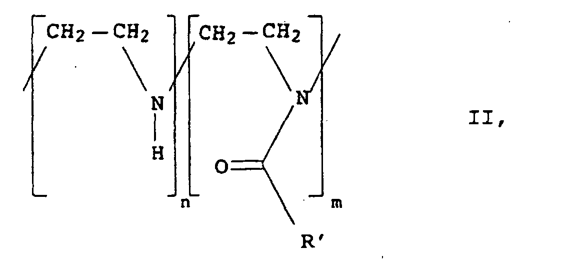 in der der Rest R' Alkyl mit 1 bis 29 C-Atomen bedeutet, die Summe aus n und m mindestens 10 und das Verhältnis aus m/m+n 0,01 bis 1 beträgt.

Weitere in Betracht kommende Liganden sind Polyethylenimine, bestehend im wesentlichen aus Einheiten der Formel III oder verzweigter Isomere davon 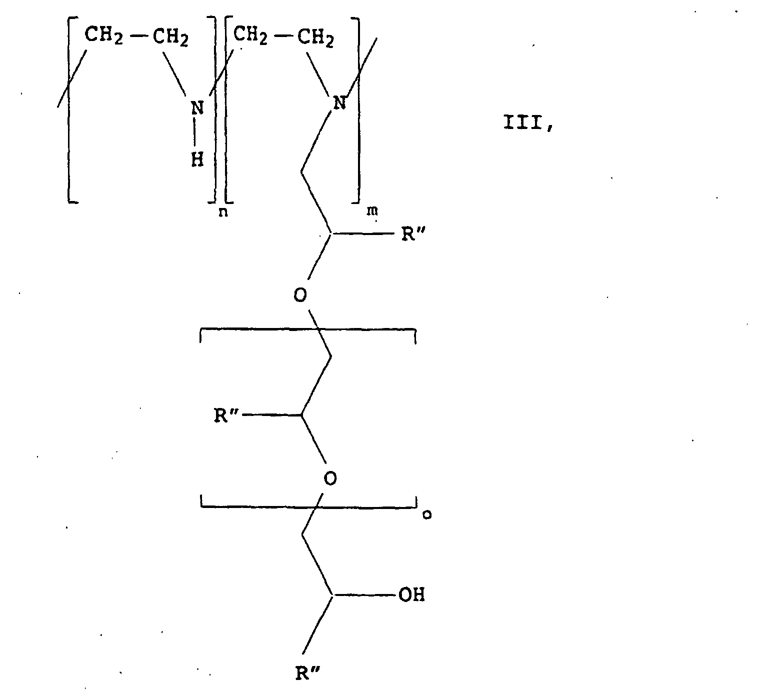 in der R" Wasserstoff oder C₁- bis C₄-Alkyl bedeutet, die Summe aus n und m mindestens 10 und das Verhältnis aus m/m+n 0,01 bis 1 beträgt und o einen Wert von 0 und 500 betragen kann. Diese Verbindungen werden als alkoxylierte Polyethylenimine bezeichnet und sind in DE 44 35 688 und DE 22 27 546 beschrieben. Auf die Angaben dieser Vorliteratur, insbesondere in Bezug auf die Herstellung der Polyamine, wird ausdrücklich Bezug genommen und deren Angaben dazu sollen als Bestandteil der vorliegenden Anmeldung gelten.

Die in den obigen Formeln I bis III gezeigten Strukturen sind idealisierte Formeln für den Fall, daß die dargestellten Polyethylenimine linear vorliegen. Die Wiederholungseinheiten können in beliebiger, z.B. statistischer, Abfolge vorliegen. Die erfindungsgemäß zu verwendenden Polyethylenimin-Komplexliganden können auch teilweise verzweigt vorliegen und Strukturelemente der nachstehend gezeigten Art aufweisen:

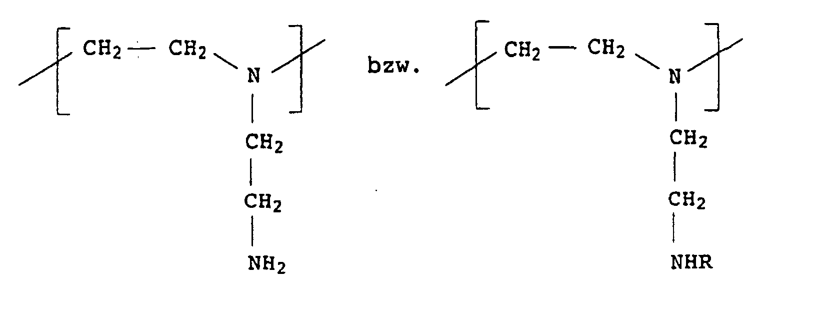

Die verzweigten Polyethylenimine sind durch das Vorhandensein primärer und tertiärer Stickstoffatome gekennzeichnet, wobei vorzugsweise höchstens 60%, bevorzugt höchstens 40% der insgesamt vorhandenen Stickstoffatome tertiäre Stickstoffatome sind.

Wenn vorliegend im Zusammenhang mit Polyethyleniminen von "verzweigten Isomeren" die Rede ist, so sind damit Konstitutionsisomere angesprochen, die sich von der dargestellten Struktur durch ein- oder mehrfaches Einfügen einer der in den Formeln I bis III in Klammern dargestellten Wiederholungseinheiten zwischen eine N-H-Bindung ableiten; sie sind über tertiäre Stickstoffatome verzweigt.

Weiter geeignet sind derivatisierte Polyvinylamine, im Wesentlichen enthaltend oder bestehend aus Einheiten der allgemeinen Formel IV 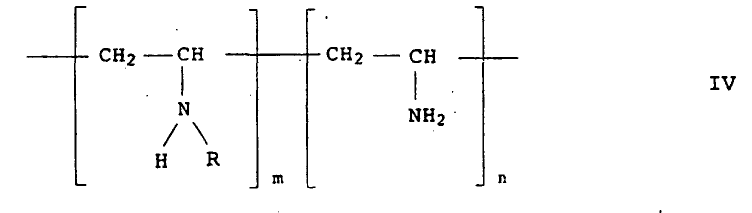 in der R, m und n die bereits angegebene Bedeutung haben.

Weiter geeignet sind derivatisierte Polyacrylamide, im Wesentlichen enthaltend oder bestehend aus Einheiten der allgemeinen Formel V 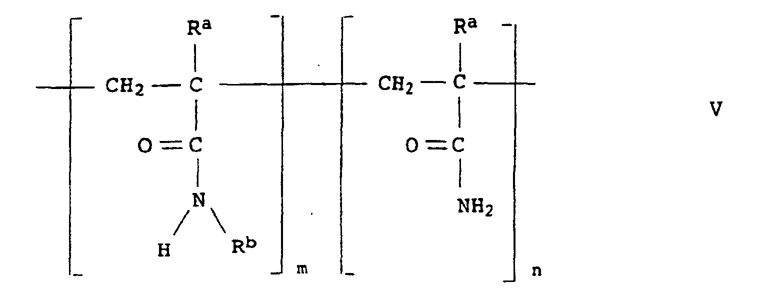 in der R^{a} für Wasserstoff oder Methyl steht, R^{b} für gleiche oder verschiedene Alkyl-, Cycloalkyl-, Aryl-, Aralkylreste mit bis zu 30 C-Atomen steht, und m und n die bereits angegebene Bedeutung haben.

Das Molekulargewicht der erfindungsgemäß zu verwendenden derivatisierten Polyaminliganden beträgt mindestens 1000 Dalton vorzugsweise mehr als 10000 Dalton. Hierbei handelt es sich um eine mittlere Molmasse, da bei der Herstellung der Polyamine und weiterer Umsetzung wie üblich eine breite Molekulargewichtsverteilung eintritt.

Im Allgemeinen sind Komplexliganden bevorzugt, die Sulfonsäuregruppenfrei sind. Weiterhin sind Komplexliganden bevorzugt, die keine Phosphan- oder Phosphingruppen tragen.

Im besonderen kommen als Komplexliganden Verbindungen, bestehend im Wesentlichen aus Einheiten der Formeln II bzw. III, oder verzweigte Isomere davon in Betracht 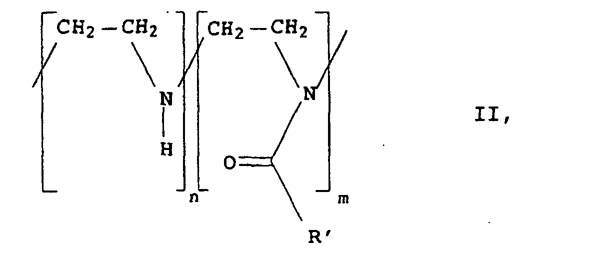 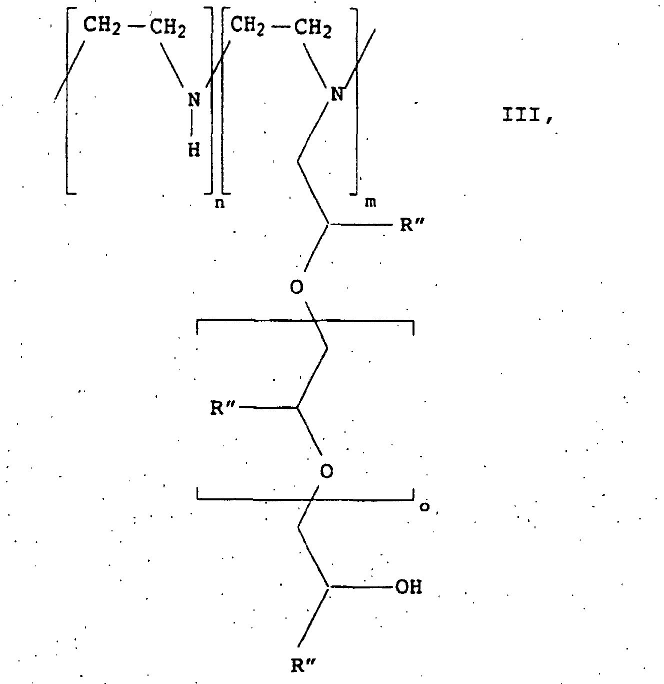 bei denen die Summe aus n + m mindestens 10 und das Verhältnis m/m+n 0,01 bis 1 und 0 einen Wert bis zu 500 beträgt und R' einen Alkylrest mit 5 bis 29 C-Atomen und R" Wasserstoff öder Methyl bedeutet.

Im einzelnen kommen z.B. in Betracht:

Umsetzungsprodukte von Homo- oder Copolymeren des Aziridins, sogenannten Polyethyleniminen (hergestellt durch Polymerisation von Aziridin), gegebenenfalls mit anderen Monomeren, wie Vinylamiden, Vinylaminen, Acrylamiden, Acrylaminen, Acrylestern, Methacrylestern und Olefinen, wie Ethen, Propen, Buten, Butadien, etc., mit einem mittleren Molekulargewicht von 200 bis 2 Millionen Dalton, mit Carbonsäuren, z.B. Ameisensäure, Essigsäure, Propionsäure, Buttersäure, Valeriansäure, Laurinsäure, 2-Ethylhexansäure, oder natürlichen C-18-Fettsäuren, wobei der Amidierungsgrad 1 bis nahe 100 %, vorzugsweise 30 bis nahe 100%, bezogen auf die amidierbaren Aminogruppen beträgt. Einzelheiten der Herstellung sind der DE-A 37 27 704 zu entnehmen.

Weiterhin Umsetzungsprodukte von Polyethyleniminen mit bis zu 500 Mol Ethylenoxid, Propylenoxid oder Butylenoxid pro Monomereinheit des Polyethylenimins, wie sie z.B. in der DE-A 44 35 688 beschrieben sind.

Unter Hydroformylierungsbedingungen bilden sich aus Rhodium enthaltenden Metallverbindungen, insbesondere Rhodiumkomplexen oder Rhodiumsalzen, und den erfindungsgemäß zu verwendenden Liganden Hydroformylierungskatalysatoren, welche Rhodium in Form von Carbonylkomplexen und assoziierten Komplexliganden enthalten.

Als zu hydroformylierende Olefine kommen beliebige Olefine mit wenigstens 7 C-Atomen, insbesondere Olefine von 7 bis 500 C-Atomen in Betracht. Die Olefine können rein oder als Gemische im erfindungsgemäßen Verfahren eingesetzt werden.

Die Olefine können geradkettig oder verzweigt sein und α-olefinische und/oder interne Doppelbindungen enthalten. Im einzelnen kommen z.B. Octen-1, Dodecen-1, Tetradecen-1, Trimer- und Tetramerpropylen, oder Dimer-, Trimer- und Tetramerbutylen in Betracht. Ebenso können ungesättigte Oligomere anderer Olefine hydroformyliert werden, desgleichen Cooligomere verschiedener Olefine. Die aus diesen Olefinen gebildeten Aldehyde dienen z.B. als Vorstufen für die Herstellung von Weichmacheralkoholen und Tensiden, die daraus auf an sich bekannte Weise durch Hydrierung erzeugt werden können. Die zur Hydroformylierung eingesetzten Olefine können durch eine Vielzahl technischer Verfahren, wie sie beispielsweise in Weissermel, Arpe: Industrielle Organische Chemie, S. 67 - 86, Verlag Chemie, Weinheim, 1994, beschrieben sind, hergestellt werden. Dazu zählen z.B. die durch gezielte Ethen-Oligomerisierung in Gegenwart von Alkylaluminiumkatalysatoren erhaltenen Ziegler-Olefine. Dazu zählen weiterhin die durch Ethen-Oligomerisierung in Gegenwart verschiedener Katalysatorsysteme erhaltenen Olefine, z.B. die in Gegenwart von Alkylaluminiumchlorid/Titantetrachlorid-Katalysatoren erhaltenen, überwiegend linearen α-Olefine und die in Gegenwart von Nickel-Phosphin-Komplex-Katalysatoren nach dem SHELL HIGHER OLEFIN PROCESS (SHOP) erhaltenen α-Olefine. Geeignete technisch zugängliche Olefingemische werden weiterhin bei der Paraffin-Dehydrierung entsprechender Erdölfraktionen, z.B. der sogenannten Petroleum- oder Dieselölfraktionen, erhalten. Zur Überführung von Paraffinen in Olefine werden im Wesentlichen das thermische Cracken (Steam Crakken), das katalytische Dehydrieren und das chemische Dehydrieren durch Chlorieren und Dehydrochlorieren eingesetzt. Geeignete Olefingemische sind weiterhin die bei Metathese- bzw. Telemerisationsreaktionen erhaltenen Olefine. Dazu zählen z.B. die Olefine aus dem Philipps-Triolefin-Prozess. Geeignete technische Olefingemische sind auch Oligomere aus dem Dimersol®-Prozess von IFP, Octol®-Prozess von Hüls, Polygas-Prozess u.s.w.

Besonders gut geeignet ist das erfindungsgemäße Verfahren auch zur Hydroformylierung von polymeren Olefinen, beispielsweise niedermolekularen Polyisobutenen, niedermolekularen Polybutadienen oder niedermolekularen 1,3-Butadien-Isobuten- oder Buten-Copolymeren. Unter "niedermolekularen Polymeren" werden insbesondere Polymere mit Molgewichten von 280 bis 5000 Dalton verstanden. Es können aber auch höhermolekulare ungesättigte Polymere mit Molgewichten von mehr als 5000 hydroformyliert werden, sofern diese unter den Reaktionsbedingungen gegebenenfalls unter Zusatz von Verdünnungsmitteln eine Phase mit dem erfindungsgemäß zu verwendenden Solubilisierungsmittel bilden.

Bei dem erfindungsgemäß zu verwendenden Solubilisierungsmittel handelt es sich um ein organisches Lösungsmittel, das so ausgewählt ist, daß es unter den Reaktionsbedingungen mit dem Olefin und dem gebildeten Hydroformylierungsprodukt eine weitgehend einheitliche Phase bildet, in welcher der Komplex solubilisiert ist, nach Veränderung bestimmter äußerer Bedingungen, wie Druck, Temperatur, An- oder Abwesenheit von Chemikalien, jedoch zu einer Phasentrennung des Reaktionsgemisches in eine solubilisierungsmittelreiche und eine solubilisierungsmittelarme Phase führt. Geeignete Lösungsmittel kann der Fachmann anhand einfacher Versuche ohne weiteres ermitteln. Vorzugsweise wird das Solubilisierungsmittel ohne Phasentransferkatalysator eingesetzt.

Das Solubilisierungsmittel liegt im Allgemeinen in der Hydroformylierungszone in einer Menge von 5 bis 75, vorzugsweise von 15 bis 60, Masse-%, bezogen auf die Gesamtmasse der in der Reaktionszone vorliegenden Substanzen (d.h. Reaktanden, Produkte und Solubilisierungsmittel) vor.

Geeignete Solubilisierungsmittel sind ausgewählt unter mehrwertigen Alkoholen, insbesondere zweiwertigen Alkoholen mit 2-12, vorzugsweise 2-6 Kohlenstoffatomen, wie Ethylenglycol, Propylenglycol;
dreiwertigen Alkoholen mit 3-12, vorzugsweise 3-6 Kohlenstoffatomen, wie Glycerin, Trimethylolpropan;
Glycolmonoalkylethern oder -dialkylethern mit 2-12, vorzugsweise 2-6 Kohlenstoffatomen in der Glycoleinheit und 1-12, vorzugsweise 1-4 Kohlenstoffatomen in den Alkylresten, wie Ethylenglycolmonomethylether, Ethylenglycolmonoethylether, Ethylenglycoldimethylether, usw.;
Alkylenoxidoligomeren und -polymeren, vorzugsweise mit einem massenmittleren Molekulargewicht von mehr als 150, insbesondere 150 bis 5000, bei denen es sich um acyclische Polyalkylenoxidpolyole oder cyclische Polyalkylenoxidether handeln kann. Vorzugsweise weisen die Oligomere ein massenmittleres Molekulargewicht im Bereich von 500 bis 2000 auf.

Geeignete acyclische Polyalkylenoxidpolyole sind unter anderem Poly(oxyalkylen)glycole, Polyalkylenoxidderivate von Glycerin (die bisweilen als Polyethertriole bezeichnet werden) sowie Polyetherpolyole mit einer Funktionalität von mehr als 3, und dergleichen. Derartige Alkohole sind unter den Bezeichnungen CARBO-WAX® PEG, CARBOWAX® TPEG, NIAX® PPG und UNCON® von der Union Carbide Corporation sowie POLYGLYCOL-E® von Dow Chemical Corporation, POLY-G® von der Olin Corporation, PLURACOL-E® von BASF, JEFFOX® von Texaco erhältlich.

Bevorzugte Polyoxyalkylenglycole sind solche der folgenden Formel 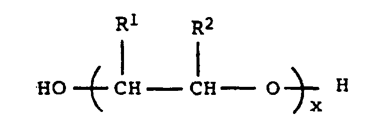 worin x für eine ganze Zahl von ≥ 2, vorzugsweise von 3 bis 30 steht und R¹ und R² unabhängig voneinander für Wasserstoff oder Methyl stehen, wie Diethylenglycol, Triethylenglycol, Tetraethylenglycol, Dipropylenglycol, Tripropylenglycol, sowie Polyethylenglycole, Polypropylenglycole oder statistische oder Blockcopolymere von Ethylenglycol und Propylenglycol mit einem mittleren Molekulargewicht von mehr als 200 g/mol. Ein Poly(oxyethylen)glycol mit einem mittleren Molekulargewicht von etwa 600 ist unter der Bezeichnung CARBOWAX® PEG-600, ein Poly(oxyethylen)glycol mit einem mittleren Molekulargewicht von etwa 150 unter der Bezeichnung CARBOWAX® PEG-150, ein Poly(oxypropylen)glycol mit einem mittleren Molekulargewicht von etwa 1025 unter der Bezeichnung NIAX® PPG-1025 erhältlich.

Bevorzugte Polyalkylenoxidderivate von Glycerin sind solche der folgenden Formel 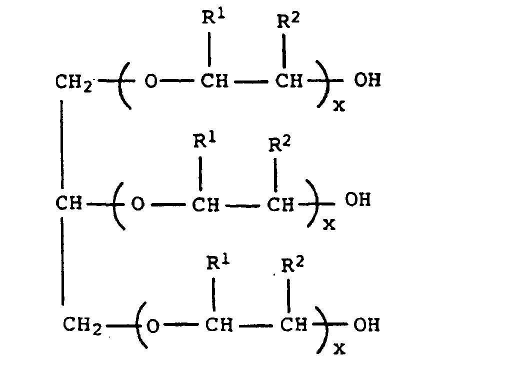 in der R¹, R² und x die oben angegebene Bedeutung haben. Die einzelnen Indices x können gleich oder verschieden sein. Ein geeigneter Vertreter ist CARBOWAX® TPEG-990, bei dem es sich um ein Polyethylenoxidderivat von Glycerin mit einem mittleren Molekulargewicht von etwa 990 handelt.

Beispielhafte cyclische Polyalkylenoxidether, die als Solubilisierungsmittel im Kontext der vorliegenden Erfindung geeignet sind, sind z.B. die in der US-A-4162261 beschriebenen Kronenether. Kronenether und Verfahren zu ihrer Herstellung sind bekannt. Vorliegend als Solubilisierungsmittel geeignete Kronenether bestehen im Wesentlichen aus Kohlenstoff, Wasserstoff und Sauerstoff und können monocyclisch oder polycyclisch vorliegen. Im Allgemeinen enthalten Kronenether im Hauptring mindestens 4 Sauerstoffatome, die voneinander durch wenigstens zwei benachbarte aliphatische Kohlenstoffatome getrennt sind. Vorzugsweise enthält der Hauptring wenigstens zwei Ringsauerstoffatome, die über Ethylen- oder substituierte Ethylengruppen miteinander verbunden sind. Die restlichen Sauerstoffatome im Hauptring sind vorzugsweise entweder an Trimethylen-, Tetramethylen-, substituierte Trimethylen- oder substituierte Tetramethylengruppen verbunden. Bevorzugte Kronenether enthalten höchstens 50, insbesondere 4 bis 15 Ethersauerstoffatome im Hauptring. Aufgrund der einfachen Herstellung sind monocyclische Kronenether bevorzugt. Beispielhafte spezifische Kronenether sind unter anderem 15-Krone-5 und 18-Krone-6, wie sie in der US-A-4162261 beschrieben sind, auf die vollinhaltlich Bezug genommen wird.

Weiterhin sind als Solubilisierungsmittel Polyglycolmonoether, oder -diether, insbesondere der folgenden Formel, geeignet, 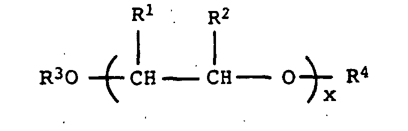 in der R¹, R² und x die oben angegebene Bedeutung haben und R³ für C₁ bis C₁₈-Alkyl oder Alkylphenyl mit 1 bis 18, vorzugsweise 4 bis 18 C-Atomen im Alkylrest und R⁴ für H oder C₁ bis C₁₈-Alkyl steht, wie Diethylenglycolmonomethylether, Diethylenglycoldimethylether, Triethylenglycolmonomethylether, usw. Bevorzugte Polyglycolmonoether weisen ein mittleres Molekulargewicht im Bereich von 300 bis 5000, insbesondere 500 bis 2000 auf. Es handelt sich z.B. um C₁ bis C₁₈-Alkoholethoxylate, -propoxylate, -butoxylate oder Mischformen davon. Derartige Alkoholalkoxylate werden durch Umsetzen eines einwertigen aliphatischen Alkohols oder eines alkylsubstituierten Phenols mit einem Alkylenoxid erhalten. Selbstverständlich kann es sich bei den einwertigen aliphatischen Alkoholen um Gemische handeln, die nach bekannten Verfahren aus Erdölabkömmlingen oder natürlichen Fetten und Ölen gewonnen werden. Geeignete Alkoholalkoxylate sind unter den Bezeichnungen TERGITOLS® von Union Carbide Corp., IGEPALS® von GAF Corp., ALFONICS® von Conoco Inc., BRIJ® von ICI, NEODOLS® von Shell Chem. Co., STANDA-MULS® von Henkel, SURFONICS® von Texaco Chem. Co., TRITONS® von Rohm & Hass Co. erhältlich. Derartige Verbindungen sind z.B. in Kirk Othmer's "Encyclopedia of Chem. Technol., 3. Aufl., Bd. 22 S. 338-9 und 364-6 (1983) beschrieben. Geeignet sind z.B. mit durchschnittlich 5 bis 8 mol Ethylenoxid ethoxylierte aliphatische C₁₂- bis C₁₅-Alkohole.

Als Solubilisierungsmittel sind weiter einwertige Alkohole mit einer Molmasse von weniger als 200 g/mol, z.B. einwertige aliphatische Alkohole mit 1 bis 12 C-Atomen, wie Methanol, Ethanol, n-Propanol, i-Propanol, Butanol, 2-Ethylhexanol, Nonanol, Octanol usw. brauchbar. Ferner sind organische Lösungsmittel mit einem Molekulargewicht von weniger als 200 g/mol und einem Hildebrand-Löslichkeitswert von 10 oder höher geeignet. Als Beispiele lassen sich aufführen (die Hildebrand-Löslichkeitsparameter sind in Klammern angegeben): Benzonitril (10,7), Acetonitril (11,8), Dimethylformamid (11,5), Dimethylacetamid (14,8), N-Methylpyrrolidon (14,8), Dimethylsulfoxid (12,8) und dergleichen. Die Hildebrand-Löslichkeitswerte sind empirische Parameter der relativen Polarität einer organischen Verbindung. Es sei z.B. auf "Introduction to Modern Liquid Chromatographie" L.R. Snyder und J.J. Kirkland, S.215-218 (1974) John Wiley & Sons, und "The Solubility of Non-Electrolytes", J.H. Hildebrand und R.L. Scott, S.424-443, Nova Publications Inc., New York (1964) verwiesen. Die genannten Solubilisierungsmittel können einzeln oder in Gemischen untereinander verwendet werden.

In bestimmten Ausführungsformen des erfindungsgemäßen Verfahrens sind Solubilisierungsmittel bevorzugt, die eine höhere Mischbarkeit mit Wasser aufweisen als das Hydroformylierungsprodukt, insbesondere wenn gemäß einem nachstehend erörterten bevorzugten Aspekt der Erfindung zur Induzierung der Phasentrennung ein Phasentrennmittel in Form von Wasser verwendet wird.

Es versteht sich, dass das Solubilisierungsmittel und der Komplexligand gegebenenfalls aufeinander abgestimmt werden müssen. Bei der Auswahl des Komplexliganden und des Solubilisierungsmittels muss außerdem die Art des zu hydroformylierenden Olefins berücksichtigt werden. Geeignete Kombinationen von Solubilisierungsmittel und Komplexliganden bei gegebenem zu hydroformylierendem Olefin kann der Fachmann anhand einfacher Versuche ohne weiteres ermitteln.

Die Hydroformylierung erfolgt in an sich bekannter Weise bei Temperaturen von 40°C bis 200°C, vorzugsweise bei 70 bis 180°C und insbesondere bei 80 bis 170°C und Drücken von 1 bis 600 bar, vorzugsweise 10 bis 400 bar und insbesondere bei 20 bis 25 bar.

Die Hydroformylierung kann in An- oder Abwesenheit organischer Lösungsmittel, die von dem erfindungsgemäß verwendeten Solubilisierungsmittel verschieden sind, durchgeführt werden, soweit diese die anschließende Phasentrennung in eine solubilisierungsmittelreiche und eine solubilisierungsmittelarme Phase nicht beeinträchtigen. Im Einzelfall kann die Mitverwendung organischer Lösungsmittel, insbesondere bei der Hydroformylierung polymerer Olefine vorteilhaft sein. Als Lösungsmittel können die bei Hydroformylierungsverfahren üblicherweise eingesetzten Lösungsmittel benutzt werden, wie hochsiedende Aldehyd-Kondensationsprodukte, die im Zuge der Hydroformylierungsreaktion als Nebenprodukt in Folge der Kondensation der Produktaldehyde entstehen. Weiter sind geeignet aromatische oder aliphatische Lösungsmittel, insbesondere Toluol, Xylol, Mihagol® und Texanol®.

Geeignete, gegebenenfalls druckfeste Reaktionsapparaturen für die Hydroformylierung sind dem Fachmann bekannt. Dazu zählen die allgemein üblichen Reaktoren für Gas/flüssig-Reaktionen, wie z.B. Rohrreaktoren, Rührkessel, Gasumlaufreaktoren, Blasensäulen etc., die gegebenenfalls durch Einbauten unterteilt sein können.

Die Hydroformylierung findet in Gegenwart von Kohlenmonoxid und Wasserstoff statt. Üblicherweise wird ein als Synthesegas bekanntes Gemisch von Kohlenmonoxid und Wasserstoff eingesetzt. Die Zusammensetzung des Synthesegases kann in weiten Bereichen variieren. Das molare Verhältnis von Kohlenmonoxid zu Wasserstoff beträgt in der Regel etwa 1:15 bis 15:1, vorzugsweise 10:1 bis 1:10, insbesondere 2:1 bis 1:2.

Der katalytisch aktive Rhodiumkomplex kann vorab hergestellt werden, wird aber zweckmäßigerweise in situ unter den Hydroformylierungsbedingungen aus einer Rhodium-Vorläuferverbindung in Gegenwart des Komplexliganden gebildet. Geeignete Rhodium-Vorläuferverbindungen sind z.B. Rhodiumdicarbonylacetylacetonat, Rh₂O₃, Rh₄(CO)₁₂, Rh₆(CO)₁₆, Salze wie Rh(NO₃)₃, RhCl₃, Rhodiumsulfat, Rhodiumcarboxylate, wie Rhodiumacetat, Rh(2-Ethylhexanoat)₃ und dergleichen.

Der Katalysator wird in der Regel in situ gebildet durch Zugabe einer Rhodium-Vorläuferverbindung und einem Überschuß z.B. 2 bis 1000 molaren Überschuß, vorzugsweise 4 bis 500-fachen und insbesondere 10 bis 100-fachen molaren Überschuß des Liganden, berechnet auf Monomereinheiten, so daß sich unter den Reaktionsbedingungen die Komplexkatalysatoren bilden.

Im übrigen wird auf einschlägige Literatur über die Hydroformylierung (z.B. Falbe loc. cit.) verwiesen.

Nach beendeter Hydroformylierungsreaktion wird in dem Reaktionsgemisch eine Phasentrennung in eine solubilisierungsmittelreiche Phase und eine solubilisierungsmittelarme Phase induziert. Der Katalysatorkomplex liegt dabei bevorzugt in der solubilisierungsmittelreichen Phase vor und das Hydroformylierungsprodukt liegt bevorzugt in der solubilisierungsmittelarmen Phase vor. Vorzugsweise enthält die solubilisierungsmittelarme Phase höchstens 10 Gew.-%, insbesondere höchstens 5 Gew.-%, und die solubilisierungsmittelreiche Phase mehr als 90 Gew.-%, insbesondere mehr als 95 Gew.-%, der zugegebenen Gesamtmenge Solubilisierungsmittel. Es ist weiterhin bevorzugt, dass die solubilisierungsmittelarme Phase mehr als 90 Gew.-%, insbesondere mehr als 95 Gew.-%, und die solubilisierungsmittelreiche Phase höchstens 10 Gew.-%, insbesondere höchstens 5 Gew.-%, der insgesamt gebildeten Hydroformylierungsprodukte enthält. Das Induzieren der Phasentrennung kann beispielsweise einen Abkühlungsschritt und/oder den Zusatz eines Phasentrennmittels umfassen. Das erfindungsgemäße Verfahren umgeht somit die Notwendigkeit, den Katalysatorkomplex bei der Aufarbeitung höheren Temperaturen als den Reaktionstemperaturen auszusetzen. Die solubilisierungsmittelreiche Phase enthält die überwiegende Menge des eingesetzten Katalysators, vorzugsweise mehr als 95 Gew.-%, insbesondere mehr als 99 Gew.-%, bezogen auf das eingesetzte Rhodium. Der Katalysator liegt in aktiver Form in der solubilisierungsmittelreichen Phase vor und kann in dieser Form für weitere Hydroformylierungen verwendet oder, in einem kontinuierlichen Hydroformylierungsverfahren, gegebenenfalls nach weiterer Behandlung, in die Reaktionszone zurückgeführt werden.

Die solubilisierungsmittelarme Phase kann neben den Hydroformylierungsprodukten gegebenenfalls unumgesetztes Olefin und/oder mit dem Ausgangsolefin eingeführte bzw. als Nebenprodukte gebildete Inerte, die der Hydroformylierung nicht zugänglich sind, enthalten. Inerte sind z.B. Paraffine, die in geringerem Umfang durch Hydrierung aus den eingesetzten Olefinen entstehen. Nach Abtrennung der Phasen voneinander kann die solubilisierungsmittelarme Phase auf an sich bekannte Weise, beispielsweise destillativ, zur Isolierung der angestrebten Wertprodukte Alkohol oder Aldehyd aufgearbeitet werden. Hierbei werden in der Regel auch Restmengen an Solubilisierungsmittel und - sofern verwendet-Phasentrennmittel entfernt. Unumgesetztes Olefin kann gegebenenfalls in die Reaktionszone zurückgeführt werden. Inerte können vor der Rückführung ausgeschleust werden.

In vielen Fällen reicht zur Induzierung der Phasentrennung in eine solubilisierungsmittelreiche und eine solubilisierungsmittelarme Phase eine Abkühlung des Reaktionsgemisches auf Temperaturen von beispielsweise unter 40°C. Im Allgemeinen beträgt die Temperaturdifferenz bei der Abkühlung wenigstens 10°C.

Alternativ oder zusätzlich kann zur Herbeiführung einer Phasentrennung dem Reaktionsgemisch ein Phasentrennmittel bzw. Entmischungsmittel zugegeben werden, das mit einer der beiden Phasen eine signifikant höhere Mischbarkeit als mit der anderen Phase aufweist. Als Phasentrennmittel sind insbesondere organische Lösungsmittel mit hoher Polarität oder Wasser geeignet. Wird das erfindungsgemäße Verfahren kontinuierlich ausgeübt, so wird dem kontinuierlich aus der Reaktionszone ausgetragenen Reaktionsgemisch vorzugsweise ein kontinuierlicher Strom des Phasentrennmittels zudosiert. Das Phasentrennmittel kann im Gleich- oder Gegenstrom zum Austrag aus der Reaktionszone geführt werden.

Die Abtrennung der Phasen voneinander kann in einer beliebigen hierzu geeigneten Vorrichtung, z.B. einem Phasenabscheider, z.B. einer Mixer-Settler-Anordnung, einer Extraktionskolonne oder anderen technischen Phasenabscheidern erfolgen. Die Phasentrennung kann unter Druck, beispielsweise unter dem Betriebsdruck der Hydroformylierungsstufe oder bei Atmosphärendruck nach vorausgegangener Entspannung des Austrags erfolgen. Die Phasentrennung kann ein- oder mehrstufig durchgeführt werden.

Bei Verwendung eines Phasentrennmittels, das eine höhere Mischbarkeit mit der solubilisierungsmittelreichen Phase aufweist und demzufolge nach der Phasentrennung in dieser vorliegt, wird das Phasentrennmittel nach erfolgter Phasentrennung vollständig oder teilweise aus der solubilisierungsmittelreichen Phase entfernt, bevor diese beispielsweise in die Reaktionszone zurückgeführt wird. Eine vollständige oder teilweise Entfernung des Phasentrennmittels und etwaiger anderer Verunreinigungen kann z.B. dadurch erfolgen, dass die solubilisierungsmittelreiche Phase oder ein Teilstrom davon einer Ultrafiltration, einer Nanofiltration, einer Destillation, einer Wasserdampfbehandlung oder einer anderen Behandlung zur Aufkonzentrierung des Katalysatorkomplexes in der solubilisierungsmittelreichen Phase unterzogen wird.

Bei großen Unterschieden der Molekulargewichte des Katalysatorkomplexes und des Solubilisierungsmittels einerseits und des Phasentrennmittels andererseits ist es mit Vorteil möglich, Katalysatorkomplex und Ligand durch Ultrafiltration in der solubilisierungsmittelreichen Phase aufzukonzentrieren. Für die Ultrafiltration kommen alle Membranen in Betracht, die gegen die in der solubilisierungsmittelreichen Phase enthaltenen Chemikalien stabil sind. Die Trenngrenze der Membranen beträgt 500 bis 200 000 Dalton. Für das Trennmembranmaterial kommen Polymere, Keramik, Glas, Metall oder Keramik auf Metall in Betracht.

Das erfindungsgemäße Verfahren kann mit Vorteil kontinuierlich durchgeführt werden. In diesem Fall kann es vorteilhaft sein, kontinuierlich oder absatzweise einen Teil der solubilisierungsmittelreichen Phase auszuschleusen und frischen Komplexliganden und/oder Rhodiumkatalysator und/oder Rhodiumverbindung und/oder Soiubilisierungsmittel kontinuierlich oder absatzweise zu ergänzen.

Die Erfindung wird nun durch die folgenden Beispiele näher veranschaulicht.

### Beispiel 1: Synthese des Komplexliganden Polyethylenimin-Propionat aus wasserfreiem Polymin durch Umsetzung mit 75 mol% Propionsäure

In einem 2 l-Vierhalskolben, der mit einem Rührer, einem Thermometer, einem Tropftrichter sowie einer Destillationsbrücke ausgestattet ist, werden 500 g (11,63 mol) eines Polyethylenimins vorgelegt. Das Polyethylenimin hat ein gewichtsmittleres Molekulargewicht (M_{W}) von ca. 25000 g/mol. Etwa 75% der vorhandenen Stickstoffatome können prinzipiell mittels Carbonsäuren amidiert werden, bei den restlichen 25% handelt es sich um tertiäre Amingruppen.

Bei einer Temperatur von 130°C tropft man binnen 2 h unter einem schwachen N₂-Strom 646 g (8,72 mol) Propionsäure hinzu. Anschließend erhöht man die Temperatur auf 160°C und destilliert das Reaktionswasser ab, wobei ein Teil der zugesetzten Propionsäure mitgeschleppt wird (34 g = 0,46 mol). Nach 4 h fügt man diese Menge Propionsäure wieder hinzu und erhitzt das Reaktionsgemisch für 15 h auf 180°C. Nach dem Abkühlen erhält man 964 g eines mit Propionsäure amidierten Polyethylenimins als gelb-roten Feststoff.

### Beispiel 2: Hydroformylierung von Polyisobuten

Polyisobuten (PIB) mit einem mittleren Molekulargewicht von ca. 1000 wurde in einer kontinuierlichen Apparatur hydroformyliert. Polyisobuten wurde aufgrund seiner hohen Viskosität mit 50 Gew.-% Mihagol® M (Kohlenwasserstoffgemisch der Fa. Wintershall) verdünnt eingesetzt. Von dieser Lösung wurden in eine zweistufige Kaskade aus Reaktoren mit gleichem Flüssigvolumen und Hubrührereinrichtung eine Zulaufmenge von 160 g (PIB+Mihagol) pro Liter Flüssigvolumen x Stunde eingespeist. Als Hydroformylierungskatalysator wurden 80 g pro Liter Flüssigvolumen x Stunde einer Lösung in die Reaktoren gepumpt, welche aus 120 mg/kg Rhodium (als Rhodiumdicarbonylacetylacetonat eingebracht) und 1,2% des Komplexliganden aus Beispiel 1 gelöst in Methyltriglycol bestand. Die Reaktionstemperatur betrug 150°C, der Synthesegasdruck (Kohlenmonoxid zu Wasserstoff 1:1) betrug 280 bar, die Abgasmenge betrug 50 Normliter/h. Der Gehalt an Wasser am Ausgang von Reaktor 2 betrug ca. 0,3 Gew.-%. Eine Hochdruck-Sichtzelle war mit dem Reaktionsraum verbunden und ließ die Einphasigkeit der Reaktionsmischung in den Reaktoren erkennen. Die aus den Reaktoren austretende Mischung wurde in einer zweistufigen Mixer-Settler-Batterie bei 40°C bei einer mittleren Gesamt-Verweilzeit von ca. 3 Stunden in eine PIB/Mihagol- und eine Methyltriglycol-Phase aufgetrennt. Der Gehalt an Wasser in der Methyltriglycol-Phase betrug ca. 0,7 Gew.-%. Die Ausbeute an Polyisobuten-Aldehyd und Polyisobuten-Alköhol, bestimmt über die analytischen Kennzahlen Carbonylzahl und Hydroxidzahl, betrug 82-85%, wobei das Verhältnis von Aldehyd/Alkohol ca. 10 mol/mol betrug. Anhand der Verseifungszahl wurde festgestellt, dass keine Formiatbildung erfolgte. Der Gehalt an Methyltriglycol in der PIB/Mihagol-Phase betrug im Mittel 2,5%. Das Methyltriglycol wurde durch eine anschließende Destillation abgetrennt. Der Rhodiumgehalt in der PIB/Mihagol-Phase betrug im Mittel 0,5 mg/kg PIB/Aldehyd(+Alkohol), der Gehalt an Komplexligand wurde indirekt über den Stickstoffgehalt bestimmt, dieser betrug im Mittel 12 mg N /kg PIB-Aldehyd(+Alkohol). Der PIB/Mihagol-Gehalt in der Methyltriglycol-Phase betrug im Mittel weniger als 1 Gew.-%.

### Beispiel 3: Hydroformylierung von Dimerbuten

80 g Dimerbuten wurde in Mischung mit 20 g Methyltriglycol bei einem Rhodiumgehalt von 20 mg/kg in einem Autoklaven mit Synthesegas (CO/H₂-Mischungsverhältnis 1:1) bei 280 bar und bei einer Temperatur von 150°C 5 h hydroformyliert. Der Rhodiumkatalysator war mit der Methyltriglycolphase in Form von Rhodiumdicarbonylacetylacetonat und dem in Beispiel 1 beschriebenen Komplexliganden in einem Mischungsverhältnis von Stickstoff aus dem Liganden/Rhodium von 100 mol/mol gelöst worden. Der Austrag des Autoklaven wurde mit 4 g Wasser versetzt und in einen Scheidetrichter überführt. Nach zwei Stunden wurde die Methyltriglycol-Wasser-Phase abgetrennt. Der Octen-Umsatz betrug 95%, die Aldehydselektivität betrug 84%, die Alkoholselektivität betrug 14%. Der Rhodiumgehalt in der Produktphase betrug 1,1 mg/kg, der Methyltriglycol-Gehalt betrug 3%.

### Beispiel 4: Hydroformylierung eines technischen C₁₂/C₁₄-Olefingemisches

80 g eines technischen C₁₂/C₁₄-Olefingemisches mit einem Gehalt an α-Olefinen von 87% und einem Gehalt an verzweigten und internen Olefinisomeren von 13% wurde in Mischung mit 20 g Methyltriglycol bei einem Rhodiumgehalt von 20 mg/kg in einem Autoklaven mit Synthesegas (CO/H₂-Mischungsverhältnis 1:1) bei 280 bar und bei einer Temperatur von 100°C 5 h analog Beispiel 3 hydroformyliert. Der Austrag des Autoklaven wurde ebenfalls mit 4 g Wasser versetzt und in einen Scheidetrichter überführt. Nach zwei Stunden wurde die Methyltriglycol-Wasser-Phase abgetrennt. Der Umsatz des α-C₁₂-Olefins betrug 99%, der Umsatz der internen und verzweigten C₁₂-Olefine betrug 98%, die C₁₃-Aldehyd+Alkohol-Selektivität betrug 97%, der Umsatz des α-C₁₄-Olefins betrug 99%, der Umsatz der internen und verzweigten C₁₄-Olefine betrug 97%, die C₁₅-Aldehyd+Alkohol-Selektivität betrug 96%. Der Rhodiumgehalt in der Produktphase betrug 1,0 mg/kg, der Methyltriglycol-Gehalt betrug 2%.

Aus diesem Beispiel ist zu erkennen, daß auch die internen und verzweigten Olefinisomere, welche als Beimischung in kommerziell erhältlichen α-Olefinmischungen enthalten sein können, ebenfalls vollständig umgesetzt werden können.

### Beispiel 5: Hydroformylierung von Trimerbutenen

80 g eines technischen Trimerbutens (erhalten nach dem Dimersol-Verfahren) wurden gemäß Beschreibung in Beispiel 3 umgesetzt. Die Ausbeute an C₁₃-Aldehyd und -Alkohol betrug 93%. Der Rhodiumgehalt in der Produktphase betrug 0,8 mg/kg; der Methyltriglycolgehalt betrug 1,7%.

### Beispiel 6: Hydroformylierung von Trimerpropenen

80 g eines technischen Trimerpropens wurden gemäß der Beschreibung in Beispiel 3 umgesetzt. Die Ausbeute an C₁₀-Aldehyd und -Alkohol betrug 90%. Der Rhodiumgehalt in der Produktphase betrug 0,9 mg/kg; der Methyltriglycolgehalt betrug 2,5%.

## Patentansprüche

1. Verfahren zur Hydroformylierung von Olefinen mit wenigstens 7 Kohlenstoffatomen, bei dem man
a) das Olefin mit Kohlenmonoxid und Wasserstoff in Gegenwart eines Komplexes von Rhodium und eines polymeren Komplexliganden, der mehr als 10 Stickstoffatome enthält und eine Molmasse von mehr als 1000 g/mol aufweist, sowie eines Solubilisierungsmittels bei einer Temperatur von 40 bis 200°C und einem Druck von 1 bis 600 bar in einer Reaktionszone einer Hydroformylierungsreaktion unterwirft, wobei unter den Reaktionsbedingungen das Solubilisierungsmittel mit dem Olefin und dem gebildeten Hydroformylierungsprodukt eine weitgehend einheitliche Phase bildet, in welcher der Komplex solubilisiert ist,
b) in dem Reaktionsgemisch eine Phasentrennung in eine solubilisierungsmittelreiche Phase und eine solubilisierungsmittelarme Phase induziert, wobei der Komplex bevorzugt in der solubilisierungsmittelreichen Phase vorliegt und das Hydroformylierungsprodukt bevorzugt in der solubilisierungsmittelarmen Phase vorliegt, und
c) die Phasen voneinander abtrennt.

2. Verfahren nach Anspruch 1, bei dem die solubilisierungsmittelreiche Phase ganz oder teilweise in die Reaktionszone zurückgeführt wird.

3. Verfahren nach Anspruch 1 oder 2, bei dem das Solubilisierungsmittel unter mehrwertigen Alkoholen, Glycolmonoethern oder -diethern, Alkylenoxidoligomeren oder -polymeren, Polyglycolmonoethern oder -diethern, Dimethylformamid, Dimethylacetamid, N-Methylpyrrolidon oder Gemischen davon ausgewählt ist.

4. Verfahren nach einem der vorhergehenden Ansprüche, bei dem als polymerer Komplexligand Polyethylenimine, die im Wesentlichen aus Einheiten der Formel I bestehen, oder verzweigte Isomere davon verwendet werden, 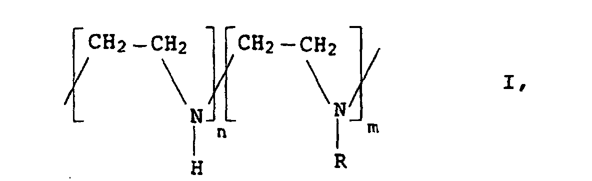 in der die Summe aus m + n mindestens 10 und das Verhältnis aus m/m+n 0,01 bis 1 beträgt und R gleiche oder verschiedene Alkyl-, Cycloalkyl-, Aryl-, Aralkyl-, Acylreste mit bis zu 30 C-Atomen oder Hydroxyalkyl(poly)oxyalkylenreste mit bis zu 500 Alkylenoxy-Einheiten bedeuten.

5. Verfahren nach einem der vorhergehenden Ansprüche, bei dem die Induzierung der Phasentrennung einen Abkühlungsschritt umfasst.

6. Verfahren nach einem der vorhergehenden Ansprüche, bei dem die Induzierung der Phasentrennung den Zusatz eines Phasentrennmittels umfasst, das mit der solubilisierungsmittelreichen Phase im Wesentlichen mischbar und mit der solubilisierungsmittelarmen Phase im Wesentlichen nicht mischbar ist.

7. Verfahren nach Anspruch 6, bei dem das Phasentrennmittel Wasser ist.

8. Verfahren nach Anspruch 6 oder 7, bei dem das Phasentrennmittel aus der solubilisierungsmittelreichen Phase zumindest teilweise entfernt wird und die solubilisierungsmittelreiche Phase in die Reaktionszone zurückgeführt wird.

9. Verfahren nach einem der vorhergehenden Ansprüche, bei dem die solubilisierungsmittelarme Phase in eine unumgesetztes Olefin enthaltende Fraktion, eine Hydroformylierungsprodukt enthaltende Fraktion und gegebenenfalls weitere Fraktionen aufgetrennt wird und die unumgesetztes Olefin enthaltende Fraktion in die Reaktionszone zurückgeführt wird.

## Claims

1. A process for the hydroformylation of olefins having at least 7 carbon atoms, in which
a) the olefin is subjected to a hydroformylation reaction with carbon monoxide and hydrogen in the presence of a complex of rhodium and a polymeric complexing ligand which contains more than 10 nitrogen atoms and has a molar mass of more than 1000 g/mol and also a solubilizer at from 40 to 200°C and a pressure of from 1 to 600 bar in a reaction zone, where the solubilizer together with the olefin and the hydroformylation product formed forms, under the reaction conditions, an essentially uniform phase in which the complex is solubilized,
b) phase separation of the reaction mixture into a solubilizer-rich phase and a phase depleted in solubilizer is induced, where the complex is preferentially present in the solubilizer-rich phase and the hydroformylation product is preferentially present in the phase depleted in solubilizer, and
c) the phases are separated from one another.

2. A process as claimed in claim 1, wherein the solubilizer-rich phase is returned completely or partly to the reaction zone.

3. A process as claimed in claim 1 or 2, wherein the solubilizer is selected from among polyhydric alcohols, glycol monoethers and glycol diethers, alkylene oxide oligomers and alkylene oxide polymers, polyglycol monoethers and polyglycol diethers, dimethylformamide, dimethylacetamide, N-methylpyrrolidone and mixtures thereof.

4. A process as claimed in any of the preceding claims, wherein the polymeric complexing ligand comprises polyethylenimines consisting essentially of units of the formula I or branched isomers thereof, 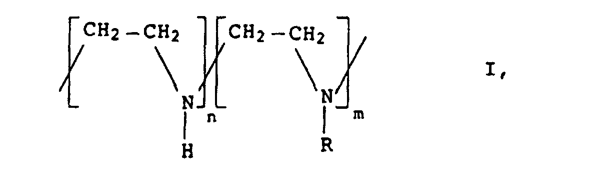 where the sum of m + n is at least 10 and the ratio m/m+n is from 0.01 to 1 and R are identical or different alkyl, cycloalkyl, aryl, aralkyl or acyl radicals having up to 30 carbon atoms or hydroxyalkyl(poly)oxyalkylene radicals having up to 500 oxyalkylene units.

5. A process as claimed in any of the preceding claims, wherein the induction of phase separation comprises a cooling step.

6. A process as claimed in any of the preceding claims, wherein the induction of the phase separation comprises addition of a phase separation agent which is essentially miscible with the solubilizer-rich phase and essentially immiscible with the phase depleted in solubilizer.

7. A process as claimed in claim 6, wherein the phase separation agent is water.

8. A process as claimed in claim 6 or 7, wherein the phase separation agent is at least partly removed from the solubilizer-rich phase and the solubilizer-rich phase is returned to the reaction zone.

9. A process as claimed in any of the preceding claims, wherein the phase depleted in solubilizer is separated into a fraction comprising unreacted olefin, a fraction comprising hydroformylation product and possibly further fractions and the fraction comprising unreacted olefin is returned to the reaction zone.

## Revendications

1. Procédé d'hydroformylation d'oléfines comportant au moins 7 atomes de carbone, dans lequel
a) on soumet l'oléfine à une réaction d'hydroformylation avec du monoxyde de carbone et de l'hydrogène, en présence d'un complexe de rhodium et d'un ligand polymère complexe, qui comporte plus de 10 atomes d'azote et présente une masse molaire de plus de 1000 g/mole, ainsi que d'un agent de solubilisation, à une température de 40 à 200°C et une pression de 1 à 600 bar, dans une zone de réaction où, dans les conditions de réaction, l'agent de solubilisation forme avec l'oléfine et le produit d'hydroformylation formé une phase pratiquement unitaire, dans laquelle le complexe est solubilisé,
b) on induit dans le mélange réactionnel une séparation de phases en une phase riche en agent de solubilisation et une phase pauvre en agent de solubilisation, le complexe se trouvant de préférence dans la phase riche en agent de solubilisation et le produit d'hydroformylation de préférence dans la phase pauvre en agent de solubilisation, et
c) on sépare les phases l'une de l'autre.

2. Procédé selon la revendication 1, dans lequel la phase riche en agent de solubilisation est totalement ou partiellement recyclée dans la zone de réaction.

3. Procédé selon la revendication 1 ou 2, dans lequel l'agent de solubilisation est choisi parmi des alcools polyvalents, des monoéthers ou des diéthers de glycol, des oxydes d'alkylène oligomères ou des oxydes d'alkylène polymères, des monéthers ou des diéthers de polyglycol, du diméthylformamide, du diméthylacétamide, de la N-méthylpyrrolidone, ou leurs mélanges.

4. Procédé selon l'une des revendications qui précèdent, dans lequel on utilise comme ligand polymère complexe des polyéthylèneimines, qui sont essentiellement constituées d'unités de la formule 1, ou des isomères ramifiés de celles-ci, 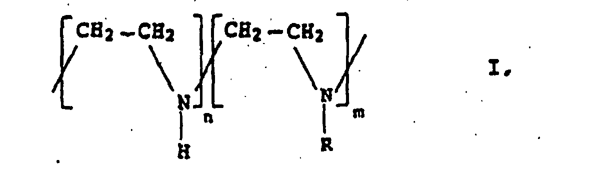 où la somme de m + n est au moins égale à 10 et le rapport de m/m+n a une valeur de 0,01 à 1 et les R sont identiques ou différents et représentent des radicaux alkyle, cycloalkyle, aryle, aralkyle, acyle comportant jusqu'à 30 atomes de C, ou des restes hydroxyalkyl(poly)oxyalkylène comportant jusqu'à 500 unités oxyalkylène.

5. Procédé selon l'une des revendications qui précèdent, dans lequel l'induction de la séparation de phases comporte une étape de refroidissement.

6. Procédé selon l'une des revendications qui précédent, dans lequel l'induction de la séparation de phases comporte l'addition d'un agent de séparation de phases, qui est essentiellement miscible à la phase riche en agent de solubilisation et qui n'est essentiellement pas miscible à la phase pauvre en agent de solubilisation.

7. Procédé selon la revendication 6, dans lequel l'agent de séparation de phases est de l'eau.

8. Procédé selon la revendication 6 ou 7, dans lequel l'agent de séparation de phases est au moins en partie éliminé de la phase riche en agent de solubilisation et la phase riche en agent de solubilisation est recyclée dans la zone de réaction.

9. Procédé selon l'une des revendications qui précèdent, dans lequel la phase pauvre en agent de solubilisation est séparée en une fraction contenant de l'oléfine non convertie, une fraction contenant le produit d'hydroformylation et éventuellement d'autres fractions, et la fraction contenant de l'oléfine non convertie est recyclée dans la zone de réaction.
